(19) Europäisches Patentamt

European Patent Office

Office européen des brevets

(11) **EP 4 715 492 A1**

(12) **EUROPEAN PATENT APPLICATION**

(43) Date of publication:
**25.03.2026 Bulletin 2026/13**

(21) Application number: **24290033.0**

(22) Date of filing: **20.09.2024**

(51) International Patent Classification (IPC):
**G05B 13/04** (2006.01)　　**G05B 13/02** (2006.01)

(52) Cooperative Patent Classification (CPC):
**G05B 13/048; G05B 13/0265;** G16C 20/30;
G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Sartorius Stedim Data Analytics AB
903 33 Umeå (SE)**

(72) Inventors:
- **Jonsson, Pär
903 33 Umeå (SE)**
- **Cloarec, Olivier
13781 Aubagne Cedex (FR)**
- **Machleid, Rafael
37079 Göttingen (DE)**

(74) Representative: **Müller-Boré & Partner
Patentanwälte PartG mbB
Friedenheimer Brücke 21
80639 München (DE)**

(54) **MONITORING AND/OR CONTROLLING A CHEMICAL AND/OR BIOLOGICAL PROCESS**

(57) A computer-implemented method is provided for monitoring and/or controlling a chemical and/or biological process. The method comprises: obtaining (S10) a time series dataset comprising observations, each one of the observations being collected with respect to the chemical and/or biological process at a particular time point, wherein each one of the observations includes values of observed parameters obtained with a spectroscopic method at the particular time point and a value of an analyte parameter obtained with a reference measurement method at the particular time point; obtaining (S20) a prediction model for estimating a predicted value of the analyte parameter in the chemical and/or biological process, the prediction model being trained using at least part of the time series dataset; validating (S30) the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model; determining (S40) whether the prediction model is valid or invalid based on a result of the validating; and monitoring and/or controlling (S50) the chemical and/or biological process when the prediction model is determined to be valid.

FIG 1

**Description**

The application relates to a computer-implemented method, computer program product and system for monitoring and/or controlling a chemical and/or biological process.

BACKGROUND

For monitoring and/or controlling chemical and/or biological processes, spectroscopic techniques have become popular as opposed to conventional techniques relying on manual sampling followed by separate offline analysis. Spectroscopic techniques can provide non-invasive measurement methods with capability of simultaneous measurement of multiple analytes, which can enable online monitoring of parameters of interest.

Adopting spectroscopic techniques for monitoring and/or controlling chemical and/or biological processes may require development and validation of calibration models for analyte parameter estimation. The calibration models may be developed and validated using multivariate regression.

SUMMARY

According to an aspect, the problem relates to facilitating improved monitoring and/or controlling of a chemical and/or biological process.

The problem is solved by the features disclosed by the independent claims. Further exemplary embodiments are defined by the dependent claims.

According to an aspect, a computer-implemented method is provided for monitoring and/or controlling a chemical and/or biological process. The method comprises:

obtaining a time series dataset comprising observations, each one of the observations being collected with respect to the chemical and/or biological process at a particular time point, wherein each one of the observations includes values of observed parameters obtained with a spectroscopic method at the particular time point and a value of an analyte parameter obtained with a reference measurement method at the particular time point;

obtaining a prediction model for estimating a predicted value of the analyte parameter in the chemical and/or biological process, the prediction model being trained using at least part of the time series dataset;

validating the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model;

determining whether the prediction model is valid or invalid based on a result of the validating; and

monitoring and/or controlling the chemical and/or biological process when the prediction model is determined to be valid.

In some exemplary embodiments, the "chemical and/or biological process" may include a biopharmaceutical drug production process. Specific examples of the chemical and/or biological process may include, but are not limited to, mammalian cell cultivations (e.g., Chinese hamster ovary (CHO) cells, human embryonic kidney (HEK) cells, etc.) for production of monoclonal antibodies (mAbs) and/or viral vectors (e.g., adeno-associated viral vectors (AAV), adenoviral vectors (AV), lentiviral vectors (LV), virus-like particle (VLP) processes, etc.).

In the present disclosure, the "observed parameters" may include parameters, values of which can be obtained (e.g., measured or calculated from one or more measured values) with a spectroscopic method. Thus, the observed parameters may represent electromagnetic spectra obtained with the spectroscopic method.

Further, in the present disclosure, the "analyte parameter" may include a parameter relating to an analyte of the chemical and/or biological process, in other words, a substance of interest in the chemical and/or biological process. For example, the analyte parameter may relate to an amount of the analyte (e.g., concentration of the analyte) that is found during and/or after the chemical and/or biological process. Specific examples of the analyte may include, but are not limited to, glucose, glutamine, glutamate, lactate, ammonium, potassium, sodium, calcium, amino acids, vitamins, titer (e.g., protein / mAb concentration), mAb glycosylations, mAb charge variants, pH, dissolved oxygen, osmolarity, viable cell concentration, viable cell count, total cell concentration, total cell count, viability, cell diameter, etc.

In the present disclosure, the "prediction model" may be a model that is configured to output a predicted value of the analyte parameter from an input including values of the observed parameters. Thus, the prediction model may be considered as a calibration model for the analyte parameter. In various aspects and exemplary embodiments described herein, the predication model may include a multivariate regression model. In some exemplary embodiments, the prediction model may include a multivariate regression model adopting partial least squares (PLS) or orthogonal partial least squares (OPLS). In other exemplary embodiments, the prediction model may involve other machine learning regression methods. For example, the prediction model may include an artificial neural network. Further examples of the machine learning regression methods may include, but are not limited to, multivariate Bayesian regression, random forest regression, ordinary least squares regression, support vector machine regression, etc.

In some exemplary embodiments, the "set of observations" used in the validating of the prediction model may be included in the time series dataset. In such exemplary embodiments, some of the observations included in the time series dataset are used for training the prediction model and other observations included in the time series dataset but are not used for training may be included in the "set of observations" used in the validating of the prediction model.

In other exemplary embodiments, the "set of observations" used in the validating of the prediction model may be observations that are not included in the time series dataset. In such exemplary embodiments, the "set of observations" may be collected in a manner analogous to those included in the time series dataset. Specifically, the set of observations may be collected with respect to the chemical and/or biological process. Each one of the set of observations may include values of the observed parameters obtained with the spectroscopic method at a particular time point and a value of the analyte parameter obtained with the reference measurement method at the particular time point.

According to any one of various aspects and exemplary embodiments described herein, since the prediction model is validated by assessing an ability of the prediction model to predict a difference between an actual value of the analyte parameter which would be obtained with the reference measurement method at a given time point and an expected value of the analyte parameter at the given time point, a prediction model which cannot predict a deviation of the value of the analyte parameter from its expected value may be considered invalid. This may lead to avoiding or reducing the risk of using a prediction model that may appear valid when assessed focusing on differences between predicted values of the analyte parameters and their expected values but has no or little capability of predicting deviations from the expected values. In other words, the likelihood of employing, for monitoring and/or controlling the chemical and/or biological process, a prediction model that is capable of predicting deviations of values of the analyte parameter from their expected values may increase with any one of various aspects and exemplary embodiments described herein. In this way, for example, in case the chemical and/or biological process is performed for producing a chemical and/or biological product (e.g., pharmaceutical drug), risks of batch failures may be reduced and/or the product quality may be increased when using a spectroscopic method for process monitoring and/or control.

Accordingly, any one of various aspects and exemplary embodiments described herein can facilitate improved monitoring and/or controlling of the chemical and/or biological method.

The method according to the above stated aspect may further comprise: outputting information indicating the prediction model to be invalid when the prediction model is determined to be invalid. This may prevent the prediction model that is determined to be invalid from being deployed for monitoring and/or controlling the chemical and/or biological process, thereby contributing to ensuring accuracy of the monitoring and/or control.

In various aspects and exemplary embodiments described herein, the assessing of the ability of the prediction model may comprise:

determining, for each observation in the set of observations, a difference between:

the value of the analyte parameter included in said observation, and
an average of values of the analyte parameter in the observations included in the at least part of the time series dataset for time points corresponding to the particular time point at which said observation is collected;

determining, for each observation in the set of observations, a difference between:

a predicted value of the analyte parameter estimated by the prediction model using the values of the observed parameters in said observation, and
an average of predicted values of the analyte parameter estimated by the prediction model using the values of the observed parameters in the observations included in at least part of the time series dataset for time points corresponding to the particular time point at which said observation is collected; and

determining a performance metric using the determined differences.

The "average of values of the analyte parameter in the observations included in the at least part of the time series dataset for time points corresponding to the particular time point at which said observation is collected" mentioned above may be considered as an expected value of the analyte parameter at the particular time point at which said observation is collected.

The "average of predicted values of the analyte parameter estimated by the prediction model using the values of the observed parameters in the observations included in at least part of the time series dataset for time points corresponding to the particular time point at which said observation is collected" mentioned above may be considered as an expected value for the predicted value of the analyte parameter at the particular time point at which said observation is collected. In some exemplary embodiments, the observations used for calculating the average of predicted values may include the observations that are included in the time series dataset and that are used for training the prediction model. Additionally or alternatively, the observations used for calculating the average of predicted values may include observations that are included in the time series dataset but are not used for training the prediction model.

The difference between the "value of the analyte parameter" and the "average of values of the analyte parameter" mentioned above may be considered as an actual difference (or deviation) of the value of the analyte parameter from the expected value. Further, the difference between the "predicted value of the analyte parameter" and the "average of predicted values of the analyte parameter" may be considered as a predicted difference (or deviation) of the value of the analyte parameter from the expected value. Thus, the "performance metric" determined using the "determined differences" may reflect the comparison between the actual and predicted deviations of the values of the analyte parameter from their expected values. Accordingly, the "performance metric" may represent the ability of the prediction model to predict deviations of values of the analyte parameter from their expected values.

In some exemplary embodiments, the determining of whether the prediction model is valid or invalid may comprise:

determining whether the performance metric meets a specified condition including a threshold that is compared with the performance metric;
determining that the prediction model is valid when the performance metric meets the specified condition; and
determining that the prediction model is invalid when the performance metric does not meet the specified condition.

The performance metric may be a root mean squared error of prediction (RMSEP), a Q2 representing goodness of prediction, or a mean absolute error of prediction. In case the RMSEP or the mean absolute error of prediction is used as the performance metric, the performance metric is determined to meet the specified condition if the performance metric is equal to or smaller than the threshold comprised in the specified condition. In case the Q2 is used as the performance metric, the performance metric is determined to meet the specified condition if the performance metric is equal to or greater than the threshold comprised in the specified condition.

In various aspects and exemplary embodiments described herein, the monitoring and/or controlling of the chemical and/or biological process may comprise:

receiving values of the observed parameters obtained with the spectroscopic method;
estimating a predicted value of the analyte parameter by inputting the received values of the observed parameters to the prediction model;
outputting the predicted value of the analyte parameter estimated by inputting the received values of the observed parameters to the prediction model; and
in case of controlling the chemical and/or biological process, controlling the chemical and/or biological process based on the outputted predicted value of the analyte parameter.

When controlling the chemical and/or biological process, one or more control signals may be generated to operate a device that performs the chemical and/or biological process. The generated control signals may be sent to the device and the device may operate according to the generated control signals. In some exemplary embodiments, the outputted predicted value of the analyte parameter may be fed into one or more feedback control loops using proportional-integral-derivative (PID) control. For example, concentrations of nutrients such as glucose and amino acids (e.g., output as the predicted values of the analyte parameters) can be adjusted with the PID control to keep conditions of the chemical and/or biopharmaceutical process within desired ranges. In such exemplary embodiments, values of one or more control variables relating to an operation of the device that performs the chemical and/or biological process may be varied according to the one or more feedback control loops of the PID control. The generated control signals as mentioned above may include instructions for the device to vary the values of the control variables in a desired manner. Examples of the control variables in the PID control may include, but are not limited to, speeds of fresh media addition and glucose solution pumps, bioreactor content removal pump speed as well as temperature and pH.

In various aspects and exemplary embodiments described herein, the validating may be performed according to cross-validation. In this case, a portion of the time series dataset may be set aside and the rest of the time series dataset may be used for training the prediction model. In the "validating" step, the set-aside portion of the time series dataset may be used as the "set of observations". The training and the "validating" step may be repeated until every observation in the time series dataset has been set aside once and used for the "validating".

In various aspects and exemplary embodiments described herein, the observations comprised in the time series data set are collected with respect to different batches of the chemical and/or biological process, each batch relating to a specified period of time including a plurality of time points.

In various aspects and exemplary embodiments described herein, the spectroscopic method may include one or more of:

nuclear magnetic resonance (NMR) spectroscopy,
Raman spectroscopy,
near-infrared (NIR) spectroscopy,
ultraviolet (UV) spectroscopy,
dielectric spectroscopy,
capacitance spectroscopy.

In various aspects and exemplary embodiments described herein, the reference measurement method may include one or more of:

liquid chromatography-mass spectrometry,
high performance liquid chromatography (HPLC) with an ultraviolet (UV) detector, a measurement method using one or more biosensors.

According to another aspect, a computer-implemented method is provided for validating a prediction model used for monitoring and/or controlling a chemical and/or biological process. The method comprises:

obtaining a time series dataset comprising observations, each one of the observations being collected with respect to the chemical and/or biological process at a particular time point, wherein each one of the observations includes values of observed parameters obtained with a spectroscopic method at the particular time point and a value of an analyte parameter obtained with a reference measurement method at the particular time point;
obtaining a prediction model for estimating a predicted value of the analyte parameter in the chemical and/or biological process, the prediction model being trained using at least part of the time series dataset; and
validating the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model.

In the method according to the other aspect stated above, the assessing may comprise:

determining, for each observation in the set of observations, a difference between:

the value of the analyte parameter included in said observation, and
an average of values of the analyte parameter in the observations included in the at least part of the time series dataset for time points corresponding to the particular time point at which said observation is collected;

determining, for each observation in the set of observations, a difference between:

a predicted value of the analyte parameter estimated by the prediction model using the values of the observed parameters in said observation, and
an average of predicted values of the analyte parameter estimated by the prediction model using the values of the observed parameters in the observations included in at least part of the time series dataset for time points corresponding to the particular time point at which said observation is collected; and determining a performance metric using the determined differences.

The method according to the other aspect stated above may further comprise:
determining whether the prediction model is valid or invalid by:

determining whether the performance metric meets a specified condition including a threshold that is compared with the performance metric,
determining that the prediction model is valid when the performance metric meets the specified condition, and
determining that the prediction model is invalid when the performance metric does not meet the specified condition.

According to yet another aspect, a computer program product is provided. The computer program product comprises computer-readable instructions that, when loaded and run on a computer, cause the computer to perform the method according to any one of the above-stated aspects and exemplary embodiments.

According to yet another aspect, a system is provided for monitoring and/or controlling a chemical and/or biological process. The system comprises:

one or more processors configured to perform the method according to any one of the above-stated aspects and exemplary embodiments; and

a storage medium configured to store the time series dataset and the set of observations,

wherein the one or more processors is or are in communication with a device configured to obtain values of the observed parameters with the spectroscopic method with respect to the chemical and/or biological process.

The subject matter described in the application can be implemented as a method or as a system, possibly in the form of one or more computer program products. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. In some examples, the system may be a general purpose computer system. In other examples, the system may be a special purpose computer system including an embedded system.

## BRIEF DESCRIPTION OF THE DRAWINGS

Details of one or more implementations are set forth in the exemplary drawings and description below. Other features will be apparent from the description, the drawings, and from the claims. It should be understood, however, that even though embodiments are separately described, single features of different embodiments may be combined to further embodiments.

**Fig. 1** shows a functional block diagram of an exemplary system according to the present disclosure.

**Fig. 2** shows examples of time trajectories for observed and predicted concentrations of glucose and potassium ions.

**Fig. 3** shows an exemplary workflow for developing and validating a prediction model.

**Fig. 4** shows a flowchart of an exemplary process for monitoring and/or controlling a chemical and/or biological process according to the present disclosure.

**Fig. 5** shows a flowchart of an example of a detailed process for validating the prediction model.

**Fig. 6** shows a flowchart of an example of a detailed process for monitoring and/or controlling the chemical and/or biological process.

**Fig. 7** shows results of experiments using a prediction model validated with a conventional validation method and with a validation method according to the present disclosure.

**Fig. 8** shows predictions of concentrations for glucose and potassium ions predicted by a prediction model.

**Fig. 9** shows an exemplary hardware configuration of a computer that may be used to implement at least a part of a system according to the present disclosure.

## DETAILED DESCRIPTION OF EMBODIMENTS

In the following text, a detailed description of examples will be given with reference to the drawings. It should be understood that various modifications to the examples may be made. In particular, one or more elements of one example may be combined and used in other examples to form new examples.

A conventional method of monitoring a chemical and/or biological process (e.g., biopharmaceutical drug production) may rely on manual sampling followed by separate offline analysis, which may cause inefficiencies. This technique may not only be time-intensive and costly, owing to a significant number of skilled staff and the heavy workload involved, but also heighten the risk of contamination and increase the likelihood of batch failures with each additional sampling step. The principal shortcoming of this traditional method may be its inherent limitation on process insights, which may be constrained by the frequency of sampling and the pace at which offline analytics can be performed. As a result, scientists may be largely left in the dark about the real-time state of the process, severely restricting their capacity to respond promptly to any deviations that may occur.

In case of biopharmaceutical drug production, for example, such limitations as stated above can have serious repercussions on product quality and may ultimately result in batch failures. With the advent of continuous production processes, a pressing need may exist for innovative solutions to bridge this information gap and enable more immediate insights into production dynamics.

In light of the above, spectroscopic techniques have been adopted for process monitoring, capitalizing on their non-invasive nature and their capability to simultaneously measure multiple analytes with the potential for inline integration. For example, the use of NMR, Raman, NIR, UV, dielectric and/or capacitance spectroscopy, in conjunction with chemometric tools, may be particularly effective for monitoring mammalian cell cultures. Spectroscopic techniques can enable the estimation of key metabolites and nutrients concentrations in cell cultures, as well as various cell and cell culture characteristics and critical quality attributes (CQAs), for example.

Furthermore, real-time monitoring of critical process parameters (CPPs) with the spectroscopic methods can facilitate the implementation of glucose feeding control loops, which may lead to enhanced productivity. Thus, the spectroscopic methods have a potential to not only monitor cell cultures in real time and in situ but also to automate processes. This advancement may even pave the way for real-time release (RTR) of batches, marking a significant leap forward in the efficiency and reliability of biopharmaceutical production. While the integration of spectroscopic techniques can achieve a significant advancement in real-time monitoring and process automation, it may be crucial to address the complexities involved in the development and validation of the underlying calibration models.

Calibration models may be developed through multivariate regression models. For instance, partial least squares (PLS) or orthogonal partial least squares (OPLS) may be applied to fit these models. However, other machine learning regression methods such as the ones involving artificial neural networks can also be employed. Further examples of the machine learning regression methods may include, but are not limited to, multivariate Bayesian regression, random forest regression, ordinary least squares regression, support vector machine regression, etc. Accuracy of predictions made by the models may be quantified and validated by, for example, a statistic metric called root mean square error of prediction (RMSEP), which can be normalized to enable comparability between different models, yielding the so called $Q2$ metric representing goodness of prediction. Typically, $Q2$ has a maximum value of 1.0, corresponding to a theoretically perfect prediction ability. Additionally or alternatively, a metric called mean absolute error of prediction may be employed.

However, in case analytes of a chemical and/or biological process in question have inherent collinearity, and/or have correlation with the progression of process time, the metrics indicating the accuracy of prediction by the models may indicate higher accuracy than they should. In other words, collinearity inherent among analytes and/or their correlation with the progression of process time may increase the likelihood of false positive results during validation (e.g., deceptively high $Q2$ or low RMSEP values). Inherent collinearity and correlation with the progression of process time may be seen, for example, among cell culture analytes such as nutrients, metabolites, and products.

False positive results of validation of the calibration models as stated above may be found, for example, when a particular spectroscopic method employed may not be physically capable of detecting a specific analyte in question. As a specific example, assume that Raman spectroscopy is employed for developing a calibration model for certain analytes within cell cultivations, where the analytes include potassium ions. Due to its measurement principle, Raman spectroscopy may not be capable of accurately detecting mononuclear ions such as potassium ions ($K^+$). However, since concentration of potassium ions in cell cultivations may have collinearity as mentioned above, the validation of the calibration model in this specific example may provide a positive result indicating that the calibration model is capable of accurately predicting concentration of the potassium ions.

Some calibration models often do not, and sometimes simply cannot, specifically target an analyte of interest. Instead, such calibration models often rely on the correlation patterns inherent in the cell culture process. Such models may lack robustness and can lead to significant prediction errors when faced with process deviations. Relying on these models for process control strategies may be risky and may even result in batch failures if the models do not perform as expected under varying conditions.

Therefore, validation methods that can detect if models were incorrectly labeled as valid may be crucial.

The present disclosure provides a method for validating a prediction model used for monitoring and/or controlling a chemical and/or biological process. The method may involve a novel approach for validating prediction models for process monitoring and/or control using spectral data from time-series. The novel approach is designed to detect the limitations of collinearity and false positives by validation techniques as described herein. A prediction model validated according to the novel approach may be employed in monitoring and/or controlling the chemical and/or biological process. The present disclosure further provides a method for monitoring and/or controlling a chemical and/or biological process.

## System Configuration

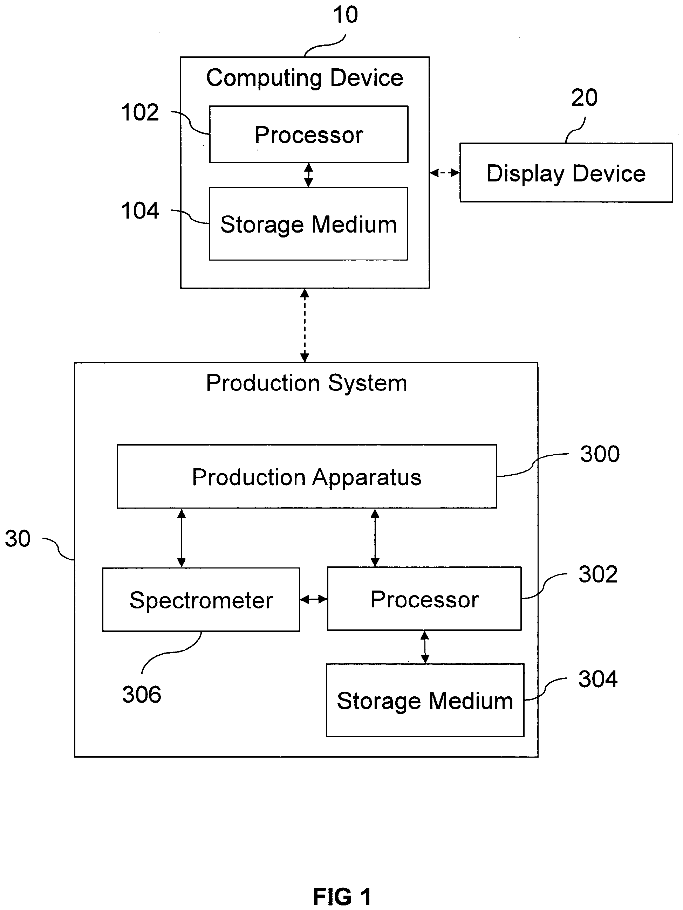

**Fig. 1** shows a functional block diagram of an exemplary system according to the present disclosure. The exemplary system shown in Fig. 1 comprises a computing device 10 and a production system 30.

The computing device 10 may be a computer connected to a display device 20 and the production system 30, via a wired connection or via one or more wired and/or wireless networks, for example. The computing device 10 may comprise a processor 102 and a storage medium 104. The processor 102 may be configured to perform one or more processes for monitoring and/or controlling a chemical and/or biological process according to the present disclosure. Details of the processes that may be performed by the processor 102 will be described later below.

The storage medium 104 may be configured to store information necessary for the processor 102 to perform the one or more processes for monitoring and/or controlling a chemical and/or biological process according to the present disclosure. Further, the storage medium 104 may be configured to store information generated and/or obtained by the processor 102.

The display device 20 may be configured to display information output by the processor 102 of the computing device 10.

The production system 30 may be a system for performing the chemical and/or biological process to be monitored and/or controlled. The production system 30 may comprise a production apparatus 300, a processor 302, storage medium 304 and a spectrometer 306. The production apparatus 300 may comprise a set of devices and/or equipment required for performing the chemical and/or biological process. For example, in case the chemical and/or biological process involves cell cultivations, the production apparatus 300 may include one or more bioreactors in which cell cultivations take place.

The processor 302 may be configured to perform one or more processes for operating the production apparatus 300. Further, the processor 302 may be configured to perform one or more processes for taking measurements of one or more parameters with respect to the chemical and/or biological process performed at the production apparatus 300. For example, the processor 302 may be configured to operate the spectrometer 306 to perform spectrometry with respect to the chemical and/or biological process. In addition, the processor 302 may be configured to transmit information relating to the chemical and/or biological process to a device (e.g., the computing device 10) external to the production system 30.

The storage medium 304 may be configured to store information necessary for the processor 302 to perform the processes for operating the production apparatus and/or for taking measurements of the one or more parameters. The storage medium 304 may further be configured to store information generated and/or obtained by the processor 302.

The spectrometer 306 may be a device configured to measure electromagnetic spectra according to a spectroscopic method. Examples of the spectroscopic method may include, but are not limited to, NMR spectroscopy, Raman spectroscopy, NIR spectroscopy, UV spectroscopy, dielectric spectroscopy and capacitance spectroscopy. The spectrometer 306 may be configured to perform spectroscopy with respect to the chemical and/or biological process performed at the production apparatus 300. The spectra measured by the spectrometer 306 may be provided to the processor 302 and the processor 302 may store the spectra in the storage medium 304.

It is noted that the system shown in Fig. 1 is merely an example and a system according to the present disclosure may have configurations different from that of the exemplary system shown in Fig. 1. For example, although Fig. 1 shows the computing device 10 and the display device 20 as two separate devices, in other exemplary systems, the computing device 10 and the display device 20 may be integrated in a single device. Further, for example, although the computing device 10 shown in Fig. 1 comprises a single processor 102, the computing device 10 may comprise more than one processor 102 and said more than one processor 102 may be configured to perform one or more processes for monitoring and/or controlling a chemical and/or biological process according to the present disclosure. In an analogous manner, the production system 30 may also comprise more than one processor 302.

Moreover, in some exemplary systems, the computing device 10 may be integrated into the production system 300. For example, the processor 302 may be further configured to perform the one or more processes for monitoring and/or controlling the chemical and/or biological process according to the present disclosure and the storage medium 304 may be configured to store information necessary for performing such process(es).

In addition, although not shown in Fig. 1, in some exemplary systems, the production system 30 may further include one or more devices for offline analysis of samples taken from the production apparatus 300 to take measurements of one or more parameters with respect to the chemical and/or biological process performed at the production apparatus 300. The processor 302 of the production system 30 may be further configured to operate the one or more devices for the offline analysis. The offline analysis may provide reference data for monitoring and/or controlling the chemical and/or biological process. Results of the offline analysis may be obtained by the processor 302 and stored in the storage medium 304.

## Validation Method

The method for validating a prediction model used for monitoring and/or controlling a chemical and/or biological process according to the present disclosure may involve the following considerations.

Given a machine learning method ML and a training dataset $\{X,y\}$ including observations $X$ (e.g., including values of observed parameters) and corresponding values of a target parameter (e.g., an analyte parameter) $y$, estimation of a new value $\hat{y}_*$ of the target parameter using a new multivariate observation $X_*$ for each time point may be expressed as follows:

$$\hat{y}_* = \mathrm{ML}(\mathrm{X},\mathrm{y})(\mathrm{X}_*) \qquad (1),$$

where each observation $X$ in the training dataset may include values of observed parameters obtained at a particular time point and the corresponding value of the target parameter $y$ may have been obtained at the particular time point. In some circumstances, the observations $X$ in the training dataset $\{X, y\}$ may have been collected with respect to different batches of a chemical and/or biological process, where each batch relates to a specified period of time including a plurality of time points.

In many machine learning methods, the prediction corresponds to a deviation $\Delta y_*$ for an observation from the mean $\bar{y}$ of the target parameter. For example, for PLS-based multivariate calibration, the first step may be the mean-centering of the data. This may mean that, in the prediction step, the mean value of $y$ is added to the prediction of the model fitted on the centered data. Accordingly, for machine learning models that have a centering step prior to training, the actual estimation may provide the deviation $\Delta y_*$ and not the estimation of new value $\hat{y}_*$ :

$$\hat{y}_* = \bar{y} + \Delta y_* \qquad (2)$$

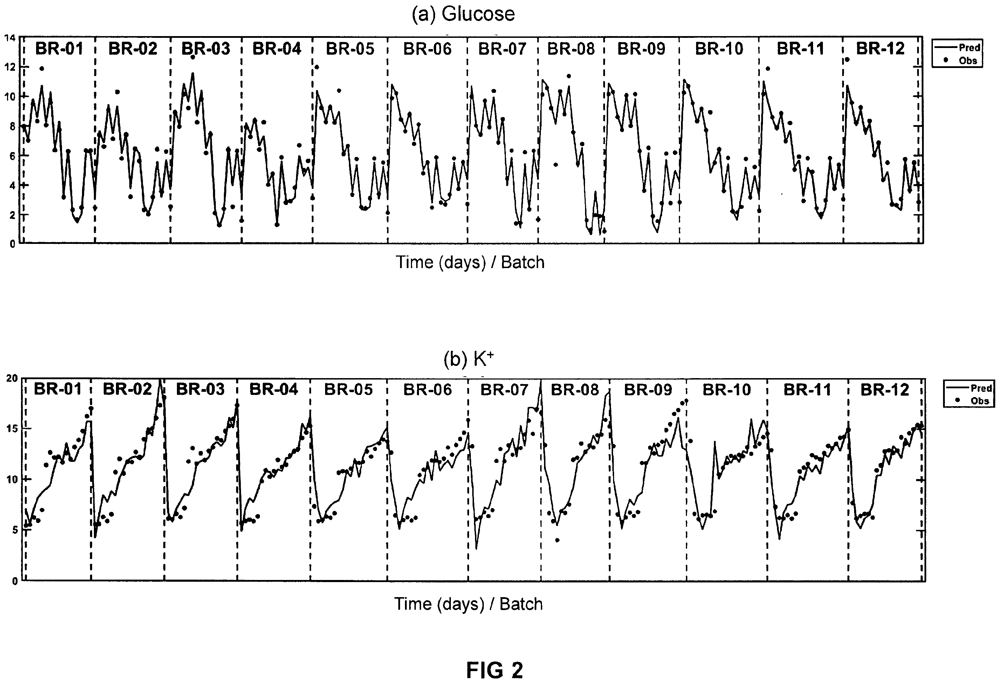

Suppose all the time point estimations are displayed on the same graph. In that case, the results of estimations may seem to provide a good estimation even though, locally, the estimation may be far from accurate and precise for a given time point. This may happen when the mean $\bar{y}_t$ of the target parameter at a given time point t is dependent on t. As specific examples, **Fig. 2** shows exemplary time trajectories for observed and predicted concentrations of Glucose and Potassium ions in a cell culture process. Fig. 2(a) shows the time trajectories for glucose and Fig. 2(b) shows the time trajectories for potassium ions. In Fig. 2, the solid lines indicate the predicted values of concentration estimated by a prediction model trained using a machine learning method and the dots indicate the observed values (in other words, actual, measured values) of concentration obtained with a conventional measurement method including manual sampling and offline analysis. Further, each of Figs. 2(a) and 2(b) includes time trajectories for 12 batches of the cell culture process, each of "BR-01" to "BR-12" corresponding to a batch process performed in a bioreactor. As can be seen from Fig. 2, although the general tendency of the time trajectories of the predicted values seem to follow that of the time trajectories of the observed values, when focused on each specific time point, the predicted values are not so close to the observed values at several time points (in particular for the potassium ions shown in Fig. 2(b)).

When estimating a new value of the target parameter y from a new observation $X_*$, using a model fitted on the whole time series, the deviation $\Delta y_*$ may be estimated so that:

$$\hat{y}_* = \bar{y} + \Delta y_* \qquad (3),$$

where $\bar{y}$ may indicate a global mean for all time points and $\hat{y}_*$ may indicate the corresponding prediction. Note that the above Equation (3) is the same as the above Equation (2). However, the deviation $\Delta y_*$ may be dependent on the time point $t$ and, thus, may be expressed as $\Delta y_* = f(t, \Delta y_t)$, where $\Delta y_t$ may indicate the deviation at a given time point $t$. Further, $\Delta y_* \to \bar{y}_t - \bar{y}$ if $\Delta y_t \simeq 0$. Then the estimated value may be:

$$\hat{y}_* \simeq \bar{y}_t \qquad (4)$$

This may mean that if there is no prediction ability of the model, the expected value of $\Delta y_t$ is 0 and the expected prediction value will be the average value at time point $t$. Therefore, any correlation between time and the target parameter (in other words, a response variable of interest) may lead to misleading prediction if not assessed and validated correctly. The goal of process monitoring may be to correctly detect deviation from the expected value of a parameter of interest and not to provide a value close to the expected value.

Considering a model ML(X,y) created using time dependent process data, where observations (also referred to as "samples") used to create the model may be represented as $(X_i, y_i)$ (i = 1, 2, ..., N), the model may be validated by applying the model to new observations (or new samples) $(X_{*j}, y_{*j})$ (j = 1, 2, ..., M) and comparing the predicted values ($\hat{y}_*$ )

with the true responses ($y_*$). In some circumstances, the new samples or observations may be a part of the time dependent process data that was available for training the model but was set aside to be used in the validation (e.g., in case of cross-validation). Alternatively, the new samples or observations may be observations that were not included in the time dependent process data that was available for training the model. The comparison between the predicted values ($\widehat{y_*}$) and the true responses ($y_*$) may be summarized using metrics such as RMSEP or Q2 calculated as follows:

$$RMSEP = \sqrt{\frac{\sum_{j=1}^{M}(y_{*j}-\widehat{y_{*j}})^2}{M}} \qquad (5)$$

$$Q2 = 1 - \frac{\sum_{i=1}^{N}(y_i-\widehat{y_{cv_i}})^2}{\sum_{i=1}^{N}(y_i-\bar{y})} \qquad (6),$$

where M may be the total number of the new observations, N may be the number of the observations used to create the model, $\widehat{y_{cv_i}}$ may be a predicted value of the target parameter obtained for cross validation using $X_i$ as the input to the trained model, and $\bar{y}$ may be the mean value of the target parameter for all the observations used for training the model.

If the model is unable to predict deviations from expected values at corresponding time points, then the term $\sum(y_* - \widehat{y_*})^2$ should be related to the sum of squares of the difference between observed values and expected values at corresponding time points. If the expected value is the same for all time points, then $\widehat{y_*} \approx \overline{y_*}$ which may give Q2 $\approx$ 0. If the expected response is dependent on time, however, then the term $\Sigma(y_* - \overline{y_*})^2$ may be larger than $\sum(y_* - \widehat{y_*})^2$ and therefore Q2>>0 - the model may appear to be valid.

A validation approach employed in the method for validating a prediction model used for monitoring and/or controlling a chemical and/or biological process according to the present disclosure can assess the prediction model's ability to predict the difference between actual and expected response values. The "actual" response values may be understood as values of the target parameter *y* which would be obtained with a reference measurement method at given time points. The "expected" response values may be understood as expected values of the target parameter *y* at the given time points.

Accordingly, the first step in the validation approach may be to calculate differences ($\Delta y_*$) between actual and expected response values. Hereinafter, the differences $\Delta y_*$ may also be referred to as "actual differences". For each observation in the test set ($X_*$, $y_*$), the actual difference $\Delta y_*$ may be calculated as follows:

$$\Delta y_* = y_* - \overline{y_*} \qquad (7)$$

where $y_*$ may be the response value (in other words, the value of the target parameter) of the new observation and $\overline{y_*}$ may be a mean value of the response values at time points corresponding to a time point *t* of $y_*$ for all the batches used to calibrate the model, in other words, for all the values of the target parameter *y* in the training dataset corresponding to the time point *t*.

The second step may be to calculate differences ($\Delta\widehat{y_*}$) between actual predicted response values and expected predicted response values. The "actual" predicted response values may be understood as values of the target parameter *y* estimated by the model using values of the observed parameters in the test set ($X_*$). The "expected" predicted response values may be understood as expected values of the target parameter *y* which the model would predict for given time points. The differences ($\Delta\widehat{y_*}$) may also be understood as predicted values of $\Delta y_*$ estimated by the model. Hereinafter, the differences ($\Delta\widehat{y_*}$) may also be referred to as "predicted differences".

With respect to each observation in the test set ($X_*$, $y_*$), the predicted difference $\Delta\widehat{y_*}$ may be calculated as follows:

$$\Delta\widehat{y_*} = \widehat{y_*} - \widehat{\overline{y_t}} \qquad (8)$$

where $\widehat{y_*}$ may be the predicted value of the target parameter *y* estimated by the model using the multivariate observation $X_*$, and $\widehat{\overline{y_t}}$ may be a mean value of the predicted values of the target parameter *y* estimated by the model using the multivariate observations $X$ included in the training dataset (and/or other dataset available for the validation) for time points corresponding to the time point $t$ of $\widehat{y}_*$ .

If there is no linear relation between all the actual differences $\triangle y_*$ and all the predicted differences $\triangle \widehat{y}_*$ , the model may not be able to detect deviation from the expected value of the target parameter $y$ at a given time point $t$. If there is a correlation between the actual differences $\triangle y_*$ and the predicted differences $\triangle \widehat{y}_*$ , the model may be able to detect deviation from the expected value of the target parameter $y$. This shows that the model can use the deviation of a multivariate observation ($\triangle X_*$) from its expected value to predict a deviation of the response ($\triangle y_*$) from its expected value. The strength of the linear relation can be estimated using the Q2 statistic, for example.

In this validation approach, metrics such as RMSEP and Q2 may be calculated by using the actual differences $\triangle y_*$ instead of the actual values $y_*$ of the target parameter, the predicted differences $\triangle \widehat{y}_*$ instead of the predicted values $\widehat{y}_*$ of the target parameter and a mean $\overline{\triangle y_*}$ of the actual differences instead of the mean $\overline{y_*}$ of the actual values of the target parameter.

Further, in this validation approach, for example, a mean absolute error of prediction may be used as a metric instead of or in addition to the RMSEP and/or Q2 metric. The mean absolute error of prediction may be expressed as follows:

$$MAEP = \frac{\sum_{i=1}^{M}|y_{*i} - \widehat{y_{*i}}|}{M}$$

**Exemplary Workflow of Model Development and Validation**

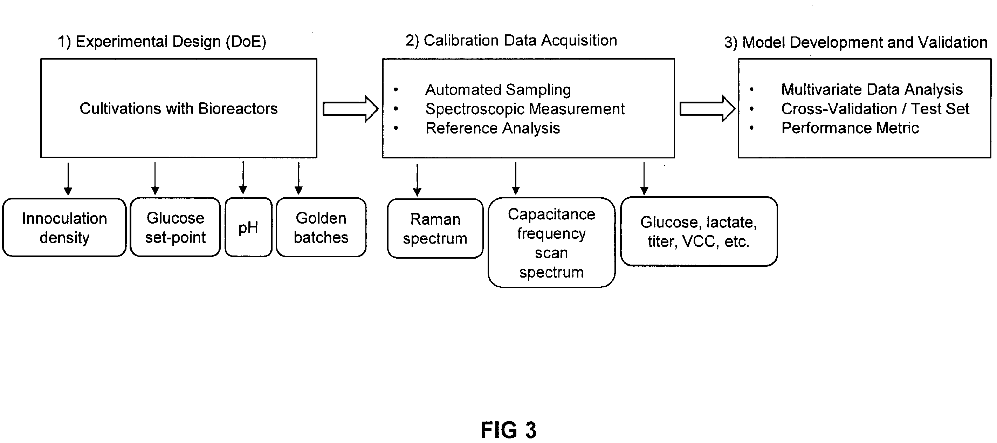

**Fig. 3** shows an exemplary workflow for developing and validating a prediction model that may be used for monitoring and/or controlling a chemical and/or biological process according to the present disclosure.

As can be seen in Fig. 3, the exemplary workflow has three stages: (A) experimental design (DoE), (B) calibration data acquisition and (C) model development and validation.

In the first stage (A), experimental design (DoE) may be carried out. Data-driven models may fail when used outside their respective training space (in other words, model extrapolation). Therefore, ideally, the training space should span as much sensible variation in critical process parameters as possible. This is typically achieved by gathering calibration data (e.g., used for training the model) from experiments planned and executed according to the DoE framework (Design of Experiments). In the specific example shown in Fig. 3, at the Experimental Design stage (A), cell cultivations are made in a plurality of bioreactors. In some (e.g., 1/2 to 3/4) of the plurality of bioreactors, process parameters such as inoculation density, glucose setpoint, pH, etc. may be varied. The rest of the plurality of bioreactors may be treated as "Golden Batches" where all the process parameters are kept within pre-defined control limits (e.g., ranges of values specified beforehand).

Once the experimental design is made in stage (A), calibration data acquisition may be performed in stage (B). Calibration model development may require at least two types of data sets to be acquired: 1) reference data from reference analytics of process samples (e.g. data obtained with a reference measurement method involving offline analysis), which is typically costly and time-consuming to produce and therefore may need to be replaced with prediction by a calibration model, and 2) spectroscopic data (e.g. Raman, NIR, etc.), typically obtained from a non-invasive, at-line/ in-line/ on-line sensor. The reference data may provide values of the analyte parameter ($y$) to be included in a training dataset used for training the calibration model. The spectroscopic data may provide values of the observed parameters ($X$) to be included in the training dataset. In some exemplary embodiments, more than one spectroscopic method may be used to obtain more than one set of spectroscopic data.

In stage (B) of Fig. 3, the chemical and/or biological process is performed according to the DoE framework determined in stage (A) to collect the reference data and spectroscopic data of one or more analyte parameters. In other words, measurements of the one or more analyte parameters with the reference measurement method and the spectroscopic method are carried out with respect to the chemical and/or biological process that is performed according to the DoE framework. In the specific example shown in Fig. 3, two kinds of spectroscopic data, Raman spectrum from Raman spectroscopy and capacitance frequency scan spectrum from capacitance spectroscopy are obtained for each sample to determine values of analyte parameters such as glucose, lactate, titer, viable cell concentration (VCC) etc. Further, in the specific example shown in Fig. 3, reference data for the analyte parameters are obtained with one or more reference measurement methods.

Table 1 below provides examples of analytes that may be monitored in the chemical and/or biological process as well as possible reference measurement methods and spectroscopic methods for the analytes. Referring to Table 1, for each analyte given in column (a), reference data may be obtained with the corresponding reference measurement method given in column (b) and spectroscopic data may be obtained with the corresponding spectroscopic method given in column (c). Column (d) indicates whether the corresponding analyte parameter given in column (a) can be directly detected with the spectroscopic method.

Table 1

| (a) Analyte (analyte parameter: y) | (b) Reference measurement method | (c) Spectroscopic method (observed parameters: X) | (d) Directly detectable with the spectroscopic method? |
|---|---|---|---|
| Glucose | Biosensor(s) | Raman / NIR | Yes / Yes |
| Glutamine | Biosensor(s) | Raman / NIR | Yes / Yes |
| Glutamate | Biosensor(s) | Raman / NIR | Yes / Yes |
| Lactate | Biosensor(s) | Raman / NIR | Yes / Yes |
| Ammonium | Biosensor(s) | Raman / NIR | Yes / Yes |
| Potassium | Biosensor(s) | Raman / NIR | No / No |
| Sodium | Biosensor(s) | Raman / NIR | No / No |
| Calcium | Biosensor(s) | Raman / NIR | No / No |
| Amino acids | LCMS | Raman / NIR | Yes / Yes |
| Vitamins | LCMS | Raman / NIR | Yes / Yes |
| Titer (protein / mAb concentration) | Biosensor(s) or HPLC-UV | Raman / NIR | Yes / Yes |
| mAb glycosylations | HPLC-UV | Raman / NIR | Yes / Yes |
| mAb charge variants | HPLC-UV | Raman / NIR | Yes / Yes |
| pH | Biosensor(s) | Raman / NIR | No / No |
| Dissolved Oxygen | Biosensor(s) | Raman / NIR | No / No |
| Osmolarity | Biosensor(s) | Raman / NIR | No / No |
| Viable Cell Concentration | Biosensor(s) | Capacitance | Yes |
| Viable Cell Count | Biosensor(s) | Capacitance | Yes |
| Total Cell Concentration | Biosensor(s) | Capacitance | No |
| Total Cell Count | Biosensor(s) | Capacitance | No |
| Viability | Biosensor(s) | Capacitance | No |
| Cell Diameter | Biosensor(s) | Capacitance | Yes |

It is noted that "LCMS" and "HPLC-UV" in Table 1 above stand for liquid chromatography-mass spectrometry and high performance liquid chromatography (HPLC) with an ultraviolet (UV) detector, respectively.

The validation method as described above may be particularly effective for the analytes that cannot be directly detected with the spectroscopic method because the calibration model may rely on the inherent collinearity and/or correlation with the progression of process time with regards to such analyte parameters for the prediction as mentioned above.

Once the calibration data is collected in stage (B), model development and validation may be performed in stage (C). With the calibration data in hand, a calibration model (e.g., the machine learning model ML in the validation method described above) can be developed. This step may be based on statistical modeling, using tools from multivariate data analysis and machine learning. Before deployment, it may be desirable that the calibration model is validated to ensure its predictive ability and quantify its prediction accuracy. This can be performed through a cross-validation approach and/or an external test set approach. In either approach, the validation may be performed to assess the prediction model's ability to predict the difference between actual and expected response values as described above in section "Validation Method". Performance metrics such as RMSECV (root mean square error of cross-validation) / RMSEP (root mean square error of prediction) or Q2 may be used to quantify a model predictive ability and determine the outcome of the validation according to some threshold. For example, one or more of the performance metrics may be calculated using the actual difference $\Delta y_*$,

the predicted difference $\Delta \hat{y}_*$ and a mean $\overline{\Delta y_*}$ as stated above in section "Validation Method".

**Exemplary Process Flows**

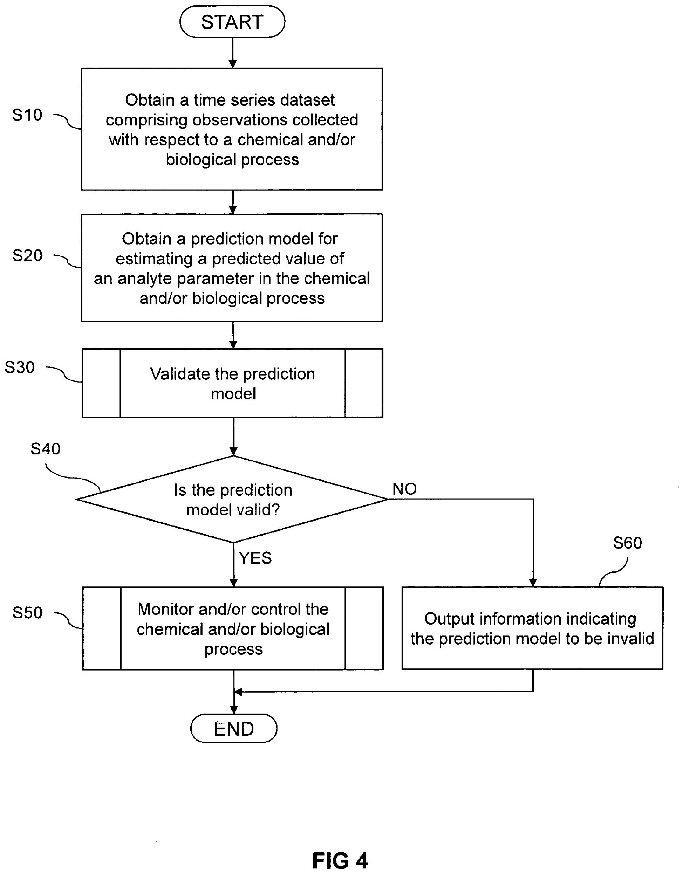

**Fig. 4** shows a flowchart of an exemplary process for monitoring and/or controlling a chemical and/or biological process according to the present disclosure. The exemplary process of Fig. 4 may be performed by the processor 102 of the computing device 10 (see Fig. 1). The exemplary process of Fig. 4 may start when the processor 102 receives a user input instructing the start of the exemplary process of Fig. 4, for example.

According to Fig. 4, at step S10, the processor 102 may obtain a time series dataset comprising observations collected with respect to a chemical and/or biological process to be monitored and/or controlled. The time series dataset may include the training dataset {X,y} referred to in the above section "Validation Method". Each one of the observations comprised in the time series dataset is collected with respect to the chemical and/or biological process at a particular time point. Each one of the observations includes values of observed parameters obtained with a spectroscopic method at the particular time point and a value of an analyte parameter obtained with a reference measurement method at the particular time point. In some exemplary embodiments, the observations comprised in the time series data set may be collected with respect to different batches of the chemical and/or biological process, each batch relating to a specified period of time including a plurality of time points. For example, each batch may relate to a period of several days, weeks or months. Further, the plurality of time points may have intervals of a certain number of minutes, hours or days. As a specific example, in each batch, the chemical and/or biological process may be performed for two weeks with measurements to obtain the observations once per day, in other words, 14 time points with a one-day interval.

The processor 102 may obtain the time series dataset at step S10 from the production system 30 (cf. Fig. 1) that performs the chemical and/or biological process. For example, the processor 302 of the production system 30 may collect the time series dataset with respect to the chemical and/or biological process performed at the production apparatus 300 and provide the collected time series dataset to the processor 102 of the computing device 10. Additionally or alternatively, the processor 102 may obtain the time series dataset from one or more devices other than the production system 30. For example, the processor 102 may access one or more databases (not shown) which stores and maintains data relating to the chemical and/or biological process in order to obtain the time series dataset.

After step S10, the exemplary process may proceed to step S20.

At step S20, the processor 102 may obtain a prediction model for estimating a predicted value of an analyte parameter in the chemical and/or biological process. The prediction model may be a model that is configured to output a predicted value of the analyte parameter ($y$) from an input ($X$) including values of the observed parameters. The prediction model may include a multivariate regression model. In some exemplary embodiments, the prediction model may include a multivariate regression model adopting PLS or OPLS. In other exemplary embodiments, the prediction model may involve other machine learning regression methods such as the ones involving an artificial neural network. Further examples of the machine learning regression methods may include, but are not limited to, multivariate Bayesian regression, random forest regression, ordinary least squares regression, support vector machine regression, etc.

The prediction model may be trained using at least part of the time series dataset obtained at step S10. In some exemplary embodiments, the processor 102 may train the prediction model at step S20 using the at least part of the time series dataset according to the machine learning method of the prediction model. In other exemplary embodiments, the processor 102 may obtain, at step S20, a prediction model that has already been trained using the at least part of the time series dataset obtained at step S10. In such exemplary embodiments, the training of the prediction model may have been performed by another computing device (not shown) that is in communication with the computing device 10. Further, in such exemplary embodiments, the processor 102 may receive information indicating which part of the time series dataset obtained at step S10 was used for the training of the prediction model. For instance, the time series dataset obtained at step S10 may be provided by the other computing device that performed the training of the prediction model together with such information.

After step S20, the exemplary process may proceed to step S30.

At step S30, the processor 102 may validate the prediction model obtained at step S20. Specifically, the processor 102 may validate the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model.

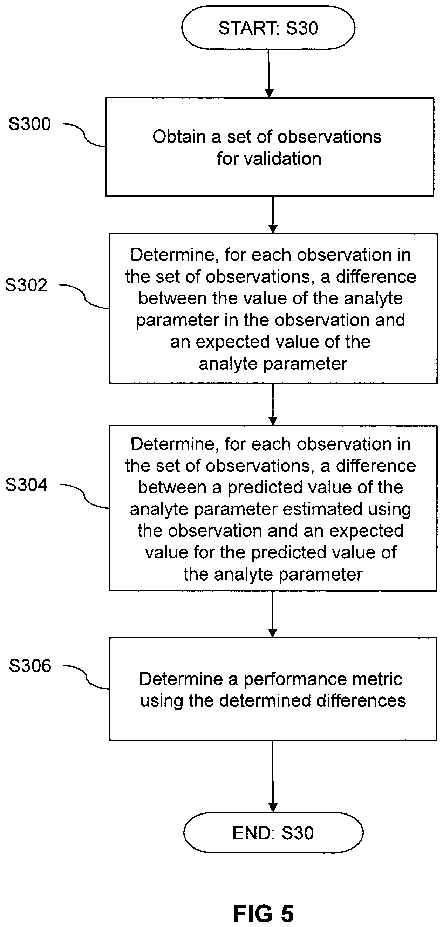

**Fig. 5** shows a flowchart of an example of a detailed process for validating the prediction model. The processor 102 may start the exemplary process shown in Fig. 5 when starting step S30 of Fig. 4.

According to Fig 5, at step S300, the processor 102 may obtain a set of observations for validation that is not included in the at least part of the time series dataset used for training the prediction model. In some exemplary embodiments, the set of observations may be a part of the time series dataset obtained at step S10 of Fig. 4, provided that the part was not used for training the prediction model. This may be the case when the validation is performed according to cross-validation. In other exemplary embodiments, the set of observations may not be included in the time series dataset obtained at step S10 of Fig. 4. In such exemplary embodiments, the set of observations may have been collected in a manner analogous to the time series dataset obtained at step S10 of Fig. 4. Similarly to the time series dataset obtained at step S10 of Fig. 4, the processor 102 may obtain the set of observations (that is not included in the time series dataset) from the production system 30 and/or one or more devices other than the production system 30.

Subsequently, at step S302, the processor 102 may determine, for each observation in the set of observations, a difference between the value of the analyte parameter in the observation and an expected value of the analyte parameter. For example, the processor 102 may calculate the difference according to Equation (7) stated in the above section "Validation Method". Accordingly, the differences determined at step S302 may be considered as the actual differences $\Delta y_*$ between actual and expected response values as mentioned in the above section "Validation Method".

After step S302, the processor 102 may determine, at step S304, for each observation in the set of observations, a difference between a predicted value of the analyte parameter estimated using the observation and an expected value for the predicted value of the analyte parameter. For example, the processor 102 may calculate the difference according to Equation (8) stated above in the above section "Validation Method". Accordingly, the differences determined at step S304 may be considered as the predicted differences $\Delta \widehat{y}_*$ between the actual predicted response values and the expected predicted response values as mentioned in the above section "Validation Method".

After step S304, the processor 102 may determine a performance metric using the determined differences at step S306. In other words, the processor 102 may use the actual differences $\Delta y_*$ determined at step S302 and the predicted differences $\Delta \widehat{y}_*$ determined at step S304 to determine the performance metric. For example, RMSEP, Q2 or a mean absolute error of prediction may be calculated as the performance metric using the actual differences $\Delta y_*$, the predicted differences $\Delta \widehat{y}_*$ and a mean $\overline{\Delta y_*}$ of the actual differences. In some exemplary embodiments, more than one performance metric may be determined at step S306.

After step S306, the exemplary process shown in Fig. 5 may end and the exemplary process may proceed to step S40 in Fig. 4.

Referring again to Fig 4, at step S40, the processor 102 may determine whether the prediction model is valid based on a result of the validation at step S30. For example, the processor 102 may determine whether the performance metric determined at step S306 of Fig. 5 meets a specified condition including a threshold that is compared with the performance metric. For example, in case the performance metric is RMSEP or a mean absolute error of prediction, the specified condition may be that the performance metric is equal to or smaller than a threshold value for the RMSEP or the mean absolute error of prediction. Further, for example, in case the performance metric is Q2, the specified condition may be that the performance metric is equal to or greater than a threshold value for the Q2. When the performance metric meets the specified condition, the prediction model may be determined to be valid. When the performance metric does not meet the specified condition, the prediction model may be determined to be invalid.

If the prediction model is determined to be valid (YES at step S40), the exemplary process may proceed to step S50.

At step S50, the processor 102 may monitor and/or control the chemical and/or biological process using the prediction model.

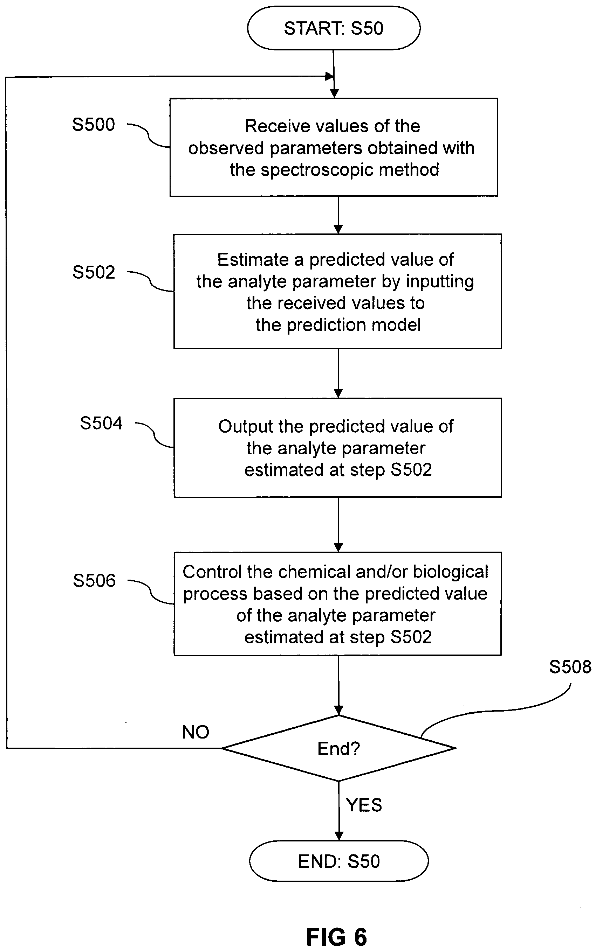

**Fig. 6** shows a flowchart of an example of a detailed process for monitoring and/or controlling the chemical and/or biological process. The processor 102 may start the exemplary process shown in Fig. 6 when starting step S50 of Fig. 4.

Referring to Fig. 6, at step S500, the processor 102 may receive values of the observed parameters obtained with the spectroscopic method with respect to the chemical and/or biological process. The spectroscopic method is the same one used for obtaining the observations included in the time series dataset obtained at step S10 of Fig. 4. The processor 102 may obtain the values of the observed parameters from the production system 30 that is performing the chemical and/or biological process, for example. More specifically, for example, the processor 302 of the production system 30 may obtain the values of the observed parameters from the spectrometer 306 while the chemical and/or biological process is being performed at the production apparatus 300 and the values of the observed parameters may be provided to the processor 102 of the computing device 10 from the processor 302 of the production system 30 (cf. Fig. 1). After step S500, the exemplary process may proceed to step S502.

At step S502, the processor 102 may estimate a predicted value of the analyte parameter by inputting the received values to the prediction model (that is determined to be valid at step S40 of Fig. 4).

Subsequently, at step S504, the processor 102 may output the predicted value of the analyte parameter estimated at step S502. For example, the processor 102 may make the display device 20 to display the predicted value. Additionally or alternatively, the processor 102 may store the predicted value in the storage medium 104. Further, in some circumstances, the processor 102 may send the predicted value to another computing device (not shown). After step S504, the exemplary process may proceed to step S506.

At step S506, the processor 102 may control the chemical and/or biological process based on the predicted value of the analyte parameter estimated at step S502. For example, the processor 102 may generate one or more control signals to operate the production apparatus 300 of the production system 30 based on the predicted value of the analyte parameter and send the generated control signals to the production system 30. The processor 302 of the production system 30 may receive the control signals and may control the production apparatus 300 according to the control signals. In some exemplary embodiments, the one or more control signals may be generated according to a feedback control loop of PID control. In such exemplary embodiments, the predicted value of the analyte parameter may be fed into the feedback control loop of the PID control and values of one or more control variables relating to an operation of the production apparatus 300 may be varied according to the feedback control loop so that the value of the analyte parameter can be kept within a desired range. The one or more control signals may include instructions for the production apparatus 300 to vary the values of the one or more control variables in a desired manner. Examples of the control variables in the PID control may include, but are not limited to, speeds of fresh media addition and glucose solution pumps, bioreactor content removal pump speed as well as temperature and pH.

It is noted that step S506 may be skipped in case of performing only the monitoring of the chemical and/or biological process.

After step S506, the processor 102 may determine, at step S508, whether to end the monitoring and/or controlling of the chemical and/or biological process. The determination may be made, for example, according to whether a stop condition is met. The stop condition may be defined by a user, for example, before the processor 102 starts the monitoring and/or controlling of the chemical and/or biological process. An example of the stop condition may be receipt of information indicating the end of the chemical and/or biological process from the production system 30. For example, when the chemical and/or biological process being performed at the production apparatus 300 of the production system 30, the processor 302 of the production system 30 may notify the end of the chemical and/or biological process to the processor 102 of the computing device 10. The processor 102 may determine to end the monitoring and/or controlling of the chemical and/or biological process at step S508 when such a notification has been received from the production system 30. Another example of the stop condition may be whether a specified period of time has passed since the start of the monitoring and/or controlling of the chemical and/or biological process. The specified period of time may be set by a user prior to the start of the monitoring and/or controlling of the chemical and/or biological process, for example. Yet another example of the stop condition may be receipt of a user input instructing the processor 102 to stop the monitoring and/or controlling of the chemical and/or biological process. In some exemplary embodiments, the stop condition may include a plurality of conditions (such as the exemplary stop conditions as mentioned above) and the processor 102 may determine that the stop condition is met when one or more of the plurality of conditions is/are met.

When the processor 102 determines not to end the monitoring and/or controlling of the chemical and/or biological process, e.g., since the stop condition is not met (NO at step S508), the exemplary process shown in Fig. 5 may return to step S500.

When the processor 102 determines to end the monitoring and/or controlling of the chemical and/or biological process, e.g., since the stop condition is met (YES at step S508), the exemplary process shown in Fig. 5 may end and step S50 of Fig. 4 may end.

Referring again to Fig. 4, if the prediction model is determined to be invalid (NO at step S40), the exemplary process may proceed to step S60.

At step S60, the processor 102 may output information indicating the prediction model to be invalid. For example, the processor 102 may make the display device 20 (cf. Fig. 1) to display the information. The prediction model determined to be invalid is preferably not used for controlling and/or monitoring the chemical and/or biological process.

After step S50 or step S60, the exemplary process shown in Fig. 4 may end.

It should be noted that the exemplary process described above with reference to Figs. 4 to 6 is merely an example and the processor 102 may perform processes different from that of Figs. 4 to 6.

For instance, in some exemplary embodiments, the processor 102 does not necessarily perform the monitoring and controlling of the chemical and/or biological process (step S50 of Fig. 4). For example, the processor 102 may simply output information indicating the prediction model to be valid instead of performing step S50 of Fig. 4 after determining that the prediction model is valid (YES at step S40). Alternatively, the processor 102 may perform steps S10 to S30 of Fig. 4 and end the process after the validation of the prediction model is made at step S30. In either case, the validated prediction model and the performance metric determined for the prediction model may be stored in the storage medium 104.

Even in the exemplary embodiments where the processor 102 ends the process after validating the prediction model without performing the monitoring and/or controlling of the chemical and/or biological process, the processor 102 may later perform monitoring and/or controlling of the chemical and/or biological process as described above with reference to step S50 of Fig. 4 and Fig. 6 using the prediction model that is determined to be valid as a result of the validation.

Further, although the exemplary process described above with reference to Figs. 4 to 6 deals with a single analyte parameter, more than one analyte parameter may be handled in an analogous manner. For example, the prediction model may be configured to output expected values of a plurality of analyte parameters and, in the validation of the prediction model, a performance metric may be determined for each of the plurality of analyte parameters according to the exemplary process shown in Fig. 5. The monitoring and/or control of the chemical and/or biological process as described above with reference to step S50 of Fig. 4 and Fig. 6 may also be performed for each of the plurality of analyte parameters.

Further, in some exemplary embodiments, two or more spectroscopic methods may be employed for collecting the time series dataset obtained at step S10 at Fig. 4. In some circumstances, data obtained with the two or more spectroscopic methods at each time point may be used as one observation obtained at said time point to predict one or more analyte parameters. In other circumstances, data obtained with different spectroscopic methods may be used for predicting different analyte parameters, where separate prediction models are developed for different analyte parameters.

The exemplary process as described above with reference to Figs. 4 to 6 and its variations may be applied to validation of multivariate models for real-time monitoring and/or control in biopharmaceutical drug production processes, for example. However, any multivariate calibration performed on data from batch or time-evolving systems can benefit from the exemplary process and its variations described above. The exemplary method and its variations described above can be applied to real-time compound concentrations and other parameter estimation using spectroscopic measurements on any type of bioreactors.

Any one of the exemplary process and its variations described above may reduce or eliminate the need of performing costly off-line measurements to invalidate potentially spurious calibration models. Further, any one of the exemplary process and its variations described above may avoid delicate calibration transfer as the calibration can be performed directly on the system involved in the measurements. This approach may work also when building the model using spiked data.

Further, the validation method used in the exemplary process and its variations may reduce the risk of using invalid models for monitoring and/or controlling the chemical and/or biological process. If invalid models are used, problems may occur at some time, for which the machine learning models such as PLS or OPLS models in general (and the software that produce the model) may be (falsely) blamed.

## Experimental Results

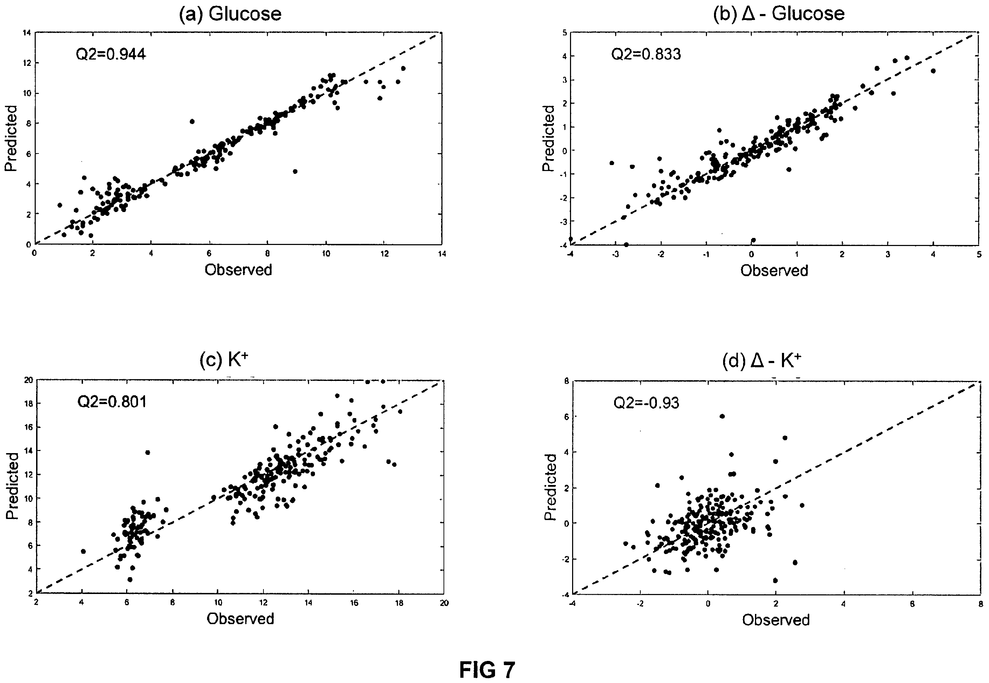

**Fig. 7** shows results of experiments using a prediction model validated with a conventional validation method and with a validation method according to the present disclosure.

In the experiments of Fig. 7, a prediction model employing the PLS regression method was trained to predict values of two analyte parameters, glucose and potassium ions, using a time series dataset obtained with respect to a cell culture process, where the spectroscopic method was Raman spectroscopy. The time series dataset was obtained from measurements using biosensors, where cell cultivations were performed in an automated miniature bioreactor with 250 ml volume. Raman spectral measurements were obtained through at-line analysis. The trained prediction model was validated with a conventional validation method and with the validation method according to the present disclosure. In the conventional validation method, a Q2 metric was calculated for each of the analyte parameters according to Equation (6) using the actual values of the analyte parameter and estimated values predicted by the prediction model. In the validation method according to the present disclosure, a Q2 metric was calculated for each of the analyte parameters using the actual differences (calculated according to Equation (7)) and the predicted differences (calculated according to Equation (8)).

Figs. 7(a) and 7(b) show the results of glucose and Figs. 7(c) and 7(d) show the results of potassium ions. Figs. 7(a) and 7(c) show the results of the conventional validation methods, where vertical axes (y-axes) represent predicted concentrations ($\widehat{y}_*$) of the respective analytes predicted by the prediction model and horizontal axes (x-axes) represent actual concentrations (y.) of the respective analytes observed (in other word, measured) with a reference method. Figs. 7(b) and 7(d) show the results of the validation method according to the present disclosure, where the vertical axes (y-axes) represent predicted deviations ($\Delta\widehat{y}_*$) from the expected concentrations of the respective analytes calculated from concentrations predicted by the prediction model and horizontal axes (x-axes) represent actual deviations ($\Delta y_*$) from the expected concentrations calculated from the observed (in other word, measured) concentrations.

As can be seen from Figs. 7(a) and 7(b), for glucose, high Q2 values are observed in both the conventional validation method and the validation method according to the present disclosure. This may indicate a causal relation between Raman spectra and the concentration of glucose. For potassium ions, on the other hand, as can be seen from Figs. 7(c) and 7(d), a high Q2 value is observed only in the conventional validation method (Fig. 7(c)). This may indicate that no causal relation exists between Raman spectra and the concentration of potassium ions.

Accordingly, with the conventional validation method, the prediction model may be considered valid because of the high Q2 values for both glucose and potassium ions. With the validation method according to the present disclosure, on the other hand, the prediction model may be considered valid only for glucose and not for potassium ions because of the high Q2 value for glucose and low Q2 value for potassium ions.

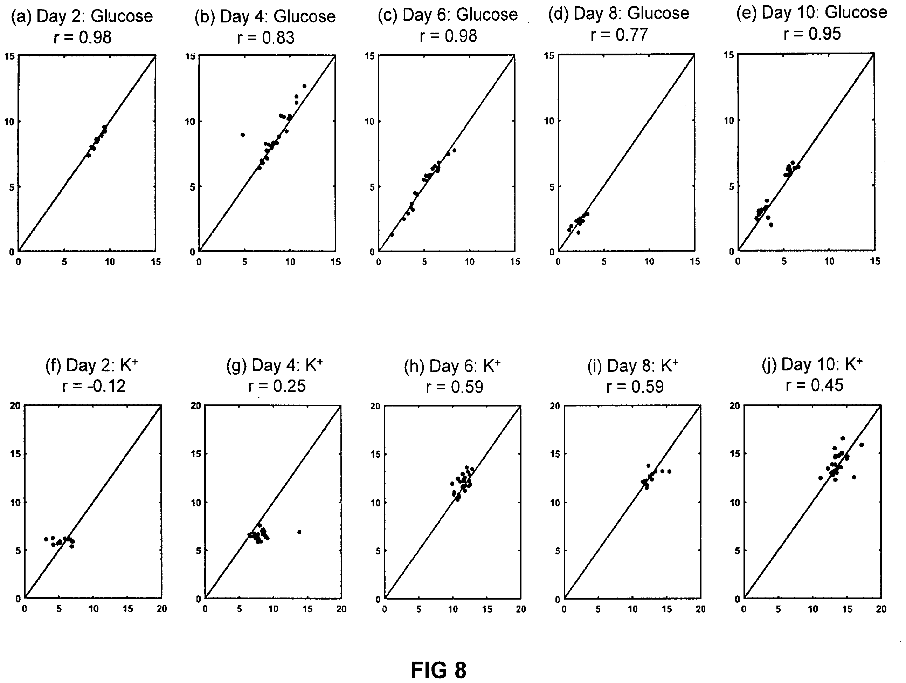

**Fig. 8** shows predictions of concentrations for glucose and potassium ions over five different days, predicted by the prediction model used in the experiments shown in Fig. 7. Figs. 8(a) to 8(e) show the predictions of glucose concentrations for day 2 (Fig. 8(a)), day 4 (Fig. 8(b)), day 6 (Fig. 8(c)), day 8 (Fig. 8(d)) and day 10 (Fig. 8(e)). Figs. 8(f) to 8(j) show the predictions of potassium ion concentrations for day 2 (Fig. 8(f)), day 4 (Fig. 8(g)), day 6 (Fig. 8(h)), day 8 (Fig. 8(i)) and day 10 (Fig. 8(j)). In Figs. 8(a) to 8(j), the vertical axes (y-axes) represent the predicted concentrations predicted by the prediction model and the horizontal axes (x-axes) represent the actual concentrations observed (in other word, measured) with a reference measurement method. The values of "r" shown in Figs. 8(a) to 8(j) indicate values of Pearson correlation coefficient for the respective predictions. As can be seen from Figs. 8(a) to 8(e), for glucose concentrations, there is a high correlation between the actual concentrations and the predicted concentrations across all days. This is not the case, however, for the potassium ion concentrations shown in Figs. 8(f) to 8(j). This difference in the correlation between the actual and predicted concentrations for glucose and potassium ions may explain the difference of the validation results for glucose and potassium ions as stated above with reference to Fig. 7.

**Hardware Configuration**

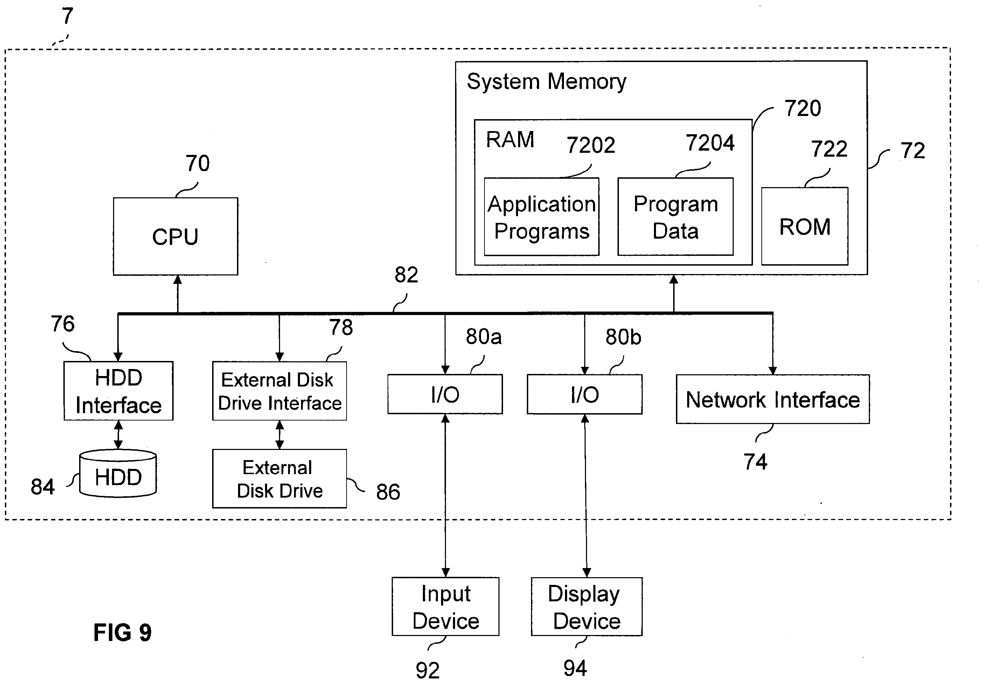

**Fig. 9** shows an exemplary hardware configuration of a computer that may be used to implement at least a part of the system as described above. For example, at least a part of the computing device 10 and/or the production system 30 shown in Fig. 1 may be implemented with the computer 7 shown in Fig. 9.

he computer 7 shown in Fig. 9 includes a central processing unit (CPU) 70, a system memory 72, a network interface 74, a hard disk drive (HDD) interface 76, an external disk drive interface 78 and input/output (I/O) interfaces 80. These components of the computer are coupled to each other via a system bus 82. The CPU 70 may perform arithmetic, logic and/or control operations by accessing the system memory 72. The system memory 72 may store information and/or instructions for use in combination with the CPU 70. The system memory 72 may include volatile and non-volatile memory, such as a random access memory (RAM) 720 and a read only memory (ROM) 722. A basic input/output system (BIOS) containing the routines that helps to transfer information between elements within the computer 7, such as during start-up, may be stored in the ROM 722. The system bus 82 may be any of several types of bus structures including a memory bus or memory controller, a peripheral bus, and a local bus using any of a variety of bus architectures.

The computer may include a network interface 74 for communicating with other computers and/or devices via a network.

Further, the computer may include a hard disk drive (HDD) 84 for reading from and writing to a hard disk (not shown), and an external disk drive 86 for reading from or writing to a removable disk (not shown). The removable disk may be a magnetic disk for a magnetic disk drive or an optical disk such as a CD ROM for an optical disk drive. The HDD 84 and the external disk drive 86 are connected to the system bus 82 by a HDD interface 76 and an external disk drive interface 78, respectively. The drives and their associated computer-readable media provide non-volatile storage of computer-readable instructions, data structures, program modules and other data for the general purpose computer. The data structures may include relevant data for the implementation of the exemplary method and its variations as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 722 or RAM 720, including an operating system (not shown), one or more application programs 7202, other program modules (not shown), and program data 7204. The application programs may include at least a part of the functionality as described above.

The computer 7 may be connected to an input device 92 such as mouse and/or keyboard and a display device 94 such as liquid crystal display, via corresponding I/O interfaces 80a and 80b as well as the system bus 82. In case the computer 7 is implemented as a tablet computer, for example, a touch panel that displays information and that receives input may be connected to the computer 7 via a corresponding I/O interface and the system bus 82. Further, in some examples, although not shown in Fig. 9, the computer 7 may further be connected to a printer and/or an imaging device such as a camera, via corresponding I/O interfaces and the system bus 82.

In addition or as an alternative to an implementation using a computer 7 as shown in Fig. 9, a part or all of the functionality of the exemplary embodiments described herein may be implemented as one or more hardware circuits. Examples of such hardware circuits may include but are not limited to: Large Scale Integration (LSI), Reduced Instruction Set Circuits (RISC), Application Specific Integrated Circuit (ASIC) and Field Programmable Gate Array (FPGA).

**Claims**

1. A computer-implemented method for monitoring and/or controlling a chemical and/or biological process, the method comprising:

   obtaining (S10) a time series dataset comprising observations, each one of the observations being collected with respect to the chemical and/or biological process at a particular time point, wherein each one of the observations includes values of observed parameters obtained with a spectroscopic method at the particular time point and a value of an analyte parameter obtained with a reference measurement method at the particular time point;
   obtaining (S20) a prediction model for estimating a predicted value of the analyte parameter in the chemical and/or biological process, the prediction model being trained using at least part of the time series dataset;
   validating (S30) the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model;
   determining (S40) whether the prediction model is valid or invalid based on a result of the validating; and
   monitoring and/or controlling (S50) the chemical and/or biological process when the prediction model is determined to be valid.

2. The method according to claim 1, further comprising:
   outputting (S60) information indicating the prediction model to be invalid when the prediction model is determined to be invalid.

3. The method according to any one of the preceding claims, wherein the assessing comprises:

   determining (S302), for each observation in the set of observations, a difference between:

      the value of the analyte parameter included in said observation, and
      an average of values of the analyte parameter in the observations included in the at least part of the time series dataset for time points corresponding to the particular time point at which said observation is collected;

   determining (S304), for each observation in the set of observations, a difference between:

      a predicted value of the analyte parameter estimated by the prediction model using the values of the observed parameters in said observation, and
      an average of predicted values of the analyte parameter estimated by the prediction model using the values of the observed parameters in the observations included in at least part of the time series dataset for time points corresponding to the particular time point at which said observation is collected; and

   determining (S306) a performance metric using the determined differences.

4. The method according to claim 3, wherein the determining of whether the prediction model is valid or invalid comprises:

   determining whether the performance metric meets a specified condition including a threshold that is compared with the performance metric;
   determining that the prediction model is valid when the performance metric meets the specified condition; and
   determining that the prediction model is invalid when the performance metric does not meet the specified condition.

5. The method according to claim 3 or 4, wherein the performance metric is a root mean squared error of prediction, RMSEP, a Q2 representing goodness of prediction, or a mean absolute error of prediction.

6. The method according to any one of the preceding claims, wherein the monitoring and/or controlling comprises:

   receiving (S500) values of the observed parameters obtained with the spectroscopic method;
   estimating (S502) a predicted value of the analyte parameter by inputting the received values of the observed parameters to the prediction model;
outputting (S504) the predicted value of the analyte parameter estimated by inputting the received values of the observed parameters to the prediction model; and
in case of controlling the chemical and/or biological process, controlling (S506) the chemical and/or biological process based on the outputted predicted value of the analyte parameter.

7. The method according to any one of the preceding claims, wherein the validating is performed according to cross-validation.

8. The method according to any one of the preceding claims, wherein the observations comprised in the time series data set are collected with respect to different batches of the chemical and/or biological process, each batch relating to a specified period of time including a plurality of time points.

9. The method according to any one of the preceding claims, wherein the spectroscopic method includes one or more of:

nuclear magnetic resonance, NMR, spectroscopy,
Raman spectroscopy,
near-infrared, NIR, spectroscopy,
ultraviolet, UV, spectroscopy,
dielectric spectroscopy,
capacitance spectroscopy.

10. The method according to any one of the preceding claims, wherein the reference measurement method includes one of more of:

liquid chromatography-mass spectrometry,
high performance liquid chromatography, HPLC, with an ultraviolet, UV, detector,
a measurement method using one or more biosensors.

11. A computer-implemented method for validating a prediction model used for monitoring and/or controlling a chemical and/or biological process, the method comprising:

obtaining (S10) a time series dataset comprising observations, each one of the observations being collected with respect to the chemical and/or biological process at a particular time point, wherein each one of the observations includes values of observed parameters obtained with a spectroscopic method at the particular time point and a value of an analyte parameter obtained with a reference measurement method at the particular time point;
obtaining (S20) a prediction model for estimating a predicted value of the analyte parameter in the chemical and/or biological process, the prediction model being trained using at least part of the time series dataset; and
validating (S30) the prediction model by assessing an ability of the prediction model to predict a difference between an actual value of the analyte parameter which would be obtained with the reference measurement method at a given time point and an expected value of the analyte parameter at the given time point, using a set of observations that is not included in the at least part of the time series dataset used for training the prediction model.

12. The method according to claim 11, wherein the assessing comprises:

determining (S302), for each observation in the set of observations, a difference between:

the value of the analyte parameter included in said observation, and
an average of values of the analyte parameter in the observations included in the at least part of the time series dataset for time points corresponding to the particular time point at which said observation is collected;

determining (S304), for each observation in the set of observations, a difference between:

a predicted value of the analyte parameter estimated by the prediction model using the values of the observed parameters in said observation, and
an average of predicted values of the analyte parameter estimated by the prediction model using the values of the observed parameters in the observations included in at least part of the time series dataset for time points corresponding to the particular time point at which said observation is collected; and

determining (S306) a performance metric using the determined differences.

13. The method according to claim 12, further comprising:
determining whether the prediction model is valid or invalid by:

determining whether the performance metric meets a specified condition including a threshold that is compared with the performance metric,
determining that the prediction model is valid when the performance metric meets the specified condition, and
determining that the prediction model is invalid when the performance metric does not meet the specified condition.

14. A computer program product comprising computer-readable instructions that, when loaded and run on a computer, cause the computer to perform the method according to any one of the preceding claims.

15. A system for monitoring and/or controlling a chemical and/or biological process, the system comprising:

one or more processors (102) configured to perform the method according to any one of claims 1 to 10; and
a storage medium (104) configured to store the time series dataset and the set of observations,
wherein the one or more processors (102) is or are in communication with a device configured to obtain values of the observed parameters with the spectroscopic method with respect to the chemical and/or biological process.

**10**

Computing Device

**102** Processor

**104** Storage Medium

**20** Display Device

**30**

Production System

**300** Production Apparatus

Spectrometer

**302** Processor

**306**

**304** Storage Medium

**FIG 1**

FIG 2

22

**FIG 3**

FIG 4

START: S30

S300 — Obtain a set of observations for validation

S302 — Determine, for each observation in the set of observations, a difference between the value of the analyte parameter in the observation and an expected value of the analyte parameter

S304 — Determine, for each observation in the set of observations, a difference between a predicted value of the analyte parameter estimated using the observation and an expected value for the predicted value of the analyte parameter

S306 — Determine a performance metric using the determined differences

END: S30

**FIG 5**

START: S50

S500 — Receive values of the
observed parameters obtained with
the spectroscopic method

S502 — Estimate a predicted value of
the analyte parameter by inputting
the received values to
the prediction model

S504 — Output the predicted value of
the analyte parameter
estimated at step S502

S506 — Control the chemical and/or biological
process based on the predicted value
of the analyte parameter
estimated at step S502

S508

NO        End?

YES

END: S50

**FIG 6**

FIG 7

(a) Day 2: Glucose  
r = 0.98

(b) Day 4: Glucose  
r = 0.83

(c) Day 6: Glucose  
r = 0.98

(d) Day 8: Glucose  
r = 0.77

(e) Day 10: Glucose  
r = 0.95

(f) Day 2: K⁺  
r = -0.12

(g) Day 4: K⁺  
r = 0.25

(h) Day 6: K⁺  
r = 0.59

(i) Day 8: K⁺  
r = 0.59

(j) Day 10: K⁺  
r = 0.45

FIG 8

EP 4 715 492 A1

**FIG 9**

Europäisches
Patentamt

European
Patent Office

Office européen
des brevets

## EUROPEAN SEARCH REPORT

**Application Number**

EP 24 29 0033

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/076318 A1 (AMGEN INC [US]) 4 May 2023 (2023-05-04) * paragraph [0004] - paragraph [0076] * ----- | 1-15 | INV. G05B13/04 G05B13/02 |
| X | WO 2020/086635 A1 (AMGEN INC [US]) 30 April 2020 (2020-04-30) * paragraph [0004] - paragraph [0035] * ----- | 1,11,14, 15 | |

**TECHNICAL FIELDS SEARCHED (IPC)**

G05B
G16C

The present search report has been drawn up for all claims

| Place of search | Date of completion of the search | Examiner |
|---|---|---|
| The Hague | 12 March 2025 | De Santis, Agostino |

EPO FORM 1503 03.82 (P04C01)

## ANNEX TO THE EUROPEAN SEARCH REPORT
## ON EUROPEAN PATENT APPLICATION NO.           EP 24 29 0033

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2025

| Patent document cited in search report | Publication date | Patent family member(s) | | Publication date |
|---|---|---|---|---|
| WO 2023076318 A1 | 04-05-2023 | AR | 127458 A1 | 24-01-2024 |
| | | AU | 2022379497 A1 | 02-05-2024 |
| | | CA | 3236194 A1 | 04-05-2023 |
| | | EP | 4423483 A1 | 04-09-2024 |
| | | JP | 2025500732 A | 15-01-2025 |
| | | TW | 202326113 A | 01-07-2023 |
| | | WO | 2023076318 A1 | 04-05-2023 |
| WO 2020086635 A1 | 30-04-2020 | AU | 2019365102 A1 | 29-04-2021 |
| | | BR | 112021007611 A2 | 27-07-2021 |
| | | CA | 3115296 A1 | 30-04-2020 |
| | | CL | 2021001024 A1 | 24-09-2021 |
| | | CN | 112912716 A | 04-06-2021 |
| | | EP | 3870957 A1 | 01-09-2021 |
| | | IL | 281977 A | 31-05-2021 |
| | | JP | 7601760 B2 | 17-12-2024 |
| | | JP | 2022512775 A | 07-02-2022 |
| | | KR | 20210078531 A | 28-06-2021 |
| | | SG | 11202103232W A | 28-05-2021 |
| | | TW | 202033949 A | 16-09-2020 |
| | | US | 2022128474 A1 | 28-04-2022 |
| | | WO | 2020086635 A1 | 30-04-2020 |

EPO FORM P0459

For more details about this annex : see Official Journal of the European Patent Office, No. 12/82